(19) Europäisches Patentamt
European Patent Office
Office européen des brevets

(11) EP 0 443 923 B1

(12) **FASCICULE DE BREVET EUROPEEN**

(45) Date de publication et mention de la délivrance du brevet: **27.08.1997 Bulletin 1997/35**

(51) Int Cl.[6]: **C07F 9/46**

(21) Numéro de dépôt: **91400401.5**

(22) Date de dépôt: **15.02.1991**

(54) **Nouveaux aminophosphine-phosphinites, leur procédé et leur application à des réactions énantiosélectives**

Neue Aminophosphin-Phosphinite, ihr Verfahren und ihre Verwendung für enantioselektive Reaktionen

New aminophosphine-phosphinites, their process and their use for enantio-selective reactions

(84) Etats contractants désignés:
**AT BE CH DE DK ES FR GB GR IT LI LU NL SE**

(30) Priorité: **19.02.1990 FR 9001976**

(43) Date de publication de la demande:
**28.08.1991 Bulletin 1991/35**

(73) Titulaire: **SANOFI**
**75008 Paris (FR)**

(72) Inventeurs:
- **Hatat, Corinne**
  **F-62660 Beuvry (FR)**
- **Roucoux, Alain**
  **F-59650 Villeneuve d'Ascq (FR)**
- **Mortreux, André**
  **F-59510 Hem (FR)**
- **Petit, Francis**
  **F-59650 Villeneuve d'Ascq (FR)**

(74) Mandataire: **Moncheny, Michel et al**
**c/o Cabinet Lavoix**
**2 Place d'Estienne d'Orves**
**75441 Paris Cedex 09 (FR)**

(56) Documents cités:
**WO-A-87/07889** **DD-A- 253 995**
**FR-A- 2 550 201**

- **TETRAHEDRON LETTERS, vol. 29, no. 16, 1988, pages 1911-1914, Pergamon Press,**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente invention concerne de nouveaux ligands phosphorés chiraux, un procédé pour leur fabrication et de nouveaux complexes de métaux de transition comportant lesdits ligands, utiles comme catalyseurs dans des réactions de synthèse énantiosélective de composés organiques.

L'intérêt de la synthèse de substances optiquement actives est bien connu, notamment dans le contexte de l'industrie pharmaceutique. De telles synthèses, ayant généralement recours à la catalyse par des complexes de métaux de transition contenant des ligands chiraux, posent le problème du choix du ligand chiral en vue d'optimiser la cinétique (en particulier la vitesse de réaction) et son rendement optique (ou excès énantiomérique). En effet, il est bien connu, en catalyse asymétrique, que les différents types de coordinats ou ligands s'avèrent spécifiques d'un composé de départ (substrat) donné.

On connaît d'après EP-A-136 210 des ligands phosphorés de formule:

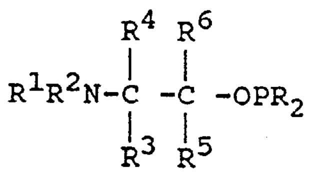

(I)

formule dans laquelle :

- R représente un radical hydrocarboné choisi parmi les radicaux alkyle, linéaires ou ramifiés, cycloalkyle ou aryle ;
- $R^1$ représente un hydrogène, un radical hydrocarboné ou un reste $-PR_2$ ;
- $R^2$ représente un hydrogène ou un radical hydrocarboné ;
- $R^3$, $R^4$, nécessairement différents, sont choisis parmi l'atome d'hydrogène et les radicaux hydrocarbonés éventuellement porteurs d'au moins une fonction choisie parmi les fonctions alcool, thiol, thiéther, amine, imine, acide, ester, amide et éther ; et
- $R^5$ et $R^6$ sont choisis parmi l'atome d'hydrogène et les radicaux hydrocarbonés éventuellement fonctionnalisés ;
- l'un des radicaux $R^3$ et $R^4$ pouvant porter une fonction $-OPR_2$ ou $-NPR_2$, $R^5$ et $R^6$ étant dans ce cas tous deux égaux à hydrogène lorsque $R^1$ représente $-PR_2$ ;
- $R^2$ et $R^3$ et les atomes d'azote et de carbone qui les portent respectivement pouvant former ensemble un hétérocycle ; ou bien $R^2$ et $R^5$, les atomes d'azote et de carbone qui les portent respectivement et l'atome de carbone intermédiaire pouvant former ensemble un hétérocycle.

On connaît également par la demande de brevet EP-A-253 700 l'utilisation de ces ligands pour l'hydrogénation asymétrique de composés carbonylés en présence d'un complexe de métal de transition de formule $MZ_q$ dans laquelle:

- M est un métal du Groupe VIII de la Classification Périodique ;
- Z représente un ou plusieurs parmi les atomes et molécules capables de complexer le métal M ; et
- q est le degré de coordination du métal M,

et, d'autre part, d'au moins un ligand phosphoré chiral.

Ce document décrit en particulier l'hydrogénation d'alpha-cétoamides et de composés alpha-dicarbonylés. Dans ce contexte, l'enseignement de ce document souffre toutefois de certaines limitations en ce qui concerne la vitesse de la réaction et son rendement optique (excès énantiomérique). Ainsi, l'hydrogénation du N-benzylbenzoylformamide conduit toujours, selon les exemples de la demande de brevet EP-A-253 700, au (S)N-benzylmandélamide avec un excès énantiomérique ne dépassant pas 86% lorsque la réaction est poursuivie pendant 6 heures à 25°C. De même, l'hydrogénation de l'oxo-2-diméthyl-3,3-butanolide-1,4 conduit toujours, selon les exemples de la demande de brevet EP-A-253 700, au (R)hydroxy-2-diméthyl-3,3-butanolide-1,4 avec un excès énantiomérique ne dépassant pas 50% lorsque la réaction est poursuivie pendant 5 heures à 25°C.

TETRAHEDRON LETTERS, vol. 29, no 16, 1988, pp1911-14, Pergamon press, Plc, GB, décrit l'hydroformylation asymétrique du styrène avec des ligands aminophosphinephosphinites.

Le problème que la présente invention vise à résoudre consiste, dans le contexte de réactions énantio-sélectives utilisant comme catalyseurs des complexes de métaux de transition contenant des ligands chiraux, et notamment dans le contexte particulier de l'hydrogénation asymétrique de composés carbonylés, à mettre au point des ligands chiraux permettant d'améliorer la vitesse et/ou le rendement optique de la réaction et/ou d'obtenir au choix l'une ou l'autre des

configurations optiques du produit de la réaction, c'est-à-dire d'améliorer qualitativement et quantitativement l'efficacité de la réaction.

Il a maintenant été découvert que l'hydrogénation asymétrique de composés carbonylés pouvait être envisagée avec d'excellents résultats grâce à l'emploi de ligands de la formule (I) précédente dans laquelle $R^2$ est un radical hydrocarboné porteur d'une fonction carbonyle.

Un premier objet de la présente invention consiste en de nouveaux ligands phosphorés chiraux comprenant au moins un radical dihydrocarbylphosphinoxy et au moins un radical dihydrocarbylphosphinamino, caractérisés en ce qu'ils sont choisis parmi ceux représentés par la formule :

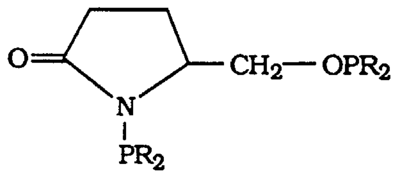

(III)

dans laquelle :

- R représente un radical hydrocarboné choisi parmi les radicaux alkyle, linéaires ou ramifiés, cycloalkyle ou aryle.

  Pour R on citera notamment :

- comme radicaux alkyle, ceux ayant de 1 à 12 atomes de carbone, par exemple les radicaux méthyle, éthyle, isopropyle, tertiobutyle et néopentyle,
- comme radicaux cycloalkyle, les radicaux cyclobutyle, cyclopentyle, cyclohexyle et norbornyle,
- comme radicaux aryle, les radicaux phényle et naphtyle.

Un second objet de la présente invention consiste en un procédé de fabrication des nouveaux ligands phosphorés chiraux décrits précédemment. Pour la préparation des ligands de formule (III), le procédé selon l'invention consiste à faire réagir dans un solvant hydrocarboné, à une température comprise entre -50°C et 80°C environ et sous atmosphère de gaz inerte le 5-oxoprolinol avec au moins un composé de formule $P(R)_2$ Y, dans laquelle Y est choisi parmi les atomes d'halogène, ledit composé étant dans un rapport molaire, relativement au 5-oxoprolinol supérieur ou égal à 2.

Par solvant hydrocarboné au sens de la présente invention, on entend par exemple un hydrocarbure aromatique tel que benzène, toluène, xylènes, éther diéthylique, tétrahydrofuranne, etc.

Y étant un atome d'halogène, pour faciliter l'élimination de l'hydracide halogéné formé par la réaction, on peut avantageusement conduire celle-ci en présence d'un excès d'amine tertiaire, telle que par exemple la triéthylamine, qui précipitera sous forme d'halogénohydrate. Le ligand phosphoré chiral est isolé successivement par filtration du précipité, suivie d'évaporation du solvant hydrocarboné sous vide. Il se présente généralement sous forme d'une huile visqueuse ou d'un solide. Le 5-oxoprolinol est un produit commercial ou facilement accessible par réductiion de l'aminoacide naturel (ou de son ester méthylique formylé).

Le 5-oxoprolinol de formule :

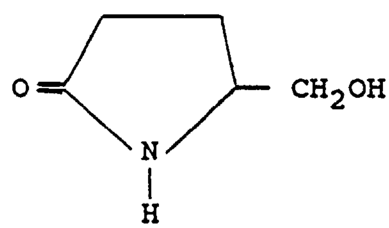

est l'aminoalcool permettant la préparation de ligands de formule (III).

Les nouveaux ligands phosphorés chiraux selon l'invention peuvent être identifiés notamment à l'aide de leurs spectres de résonance magnétique nucléaire du proton, du carbone 13 et du phosphore 31, ainsi que de leur pouvoir rotatoire spécifique. Le tableau I ci-après résume les données d'identification de certains ligands dérivés du 5-oxoprolinol en fonction de la nature des radicaux R.

Dans ce tableau $[\alpha]_D^{25}$ désigne le pouvoir rotatoire spécifique à 25°C exprimé en degrés pour la raie D du sodium et $\delta$ désigne le déplacement chimique exprimé en ppm par rapport à l'acide phosphorique (cas du phosphore 31) ou au tétraméthylsilane (cas du proton et du carbone 13). Les notations (m), (dd), (d) et (s) désignent respectivement un multiplet, un double doublet, un doublet et un singulet.

Un troisième objet de la présente invention concerne l'application des nouveaux ligands phosphorés chiraux décrits précédemment à des réactions de synthèse énantiosélective de composés organiques. Cette application consiste à faire réagir au moins un composé organique ne possédant pas de centre d'asymétrie avec (A), d'une part, au moins un complexe de métal de transition de formule MZq dans laquelle M est un métal du groupe VIII de la Classification Périodique, q est le degré de coordination du métal M et Z est un atome ou une molécule capable de complexer le métal M et, (B) d'autre part, au moins un ligand phosphoré chiral décrit précédemment. Le cas échéant, ladite réaction peut être effectuée en présence de (C) au moins un agent capable de capter au moins un ligand Z du constituant (A). Cet agent (C) peut être soit un acide possédant un anion A- peu coordinant et stériquement encombré ou un sel métallique d'un tel acide, soit une quantité d'électricité mise en jeu par électrolyse à potentiel cathodique imposé. Par anion $A^-$ peu coordinant et stériquement encombré, on entend notamment les anions perchlorate, tétrafluoro- et tétraphénylborate, hexafluorophosphate. Les sels métalliques utilisables sont principalement ceux d'argent et de thallium.

D'autre part, ladite réaction de synthèse énantiosélective peut être effectuée, le cas échéant, en présence de (D) au moins un activateur choisi parmi les dérivés aluminiques de formule $AlR_nX_{3-n}$, dans laquelle $0 \leq n \leq 3$, X est un atome d'halogène et R est un radical alkyle ayant de 1 à 12 atomes de carbone. Les constituants (A) et (B) sont généralement dans un rapport molaire (B)/(A) compris entre 1 et 10 environ. Les constituants (A) et (D) sont généralement dans un rapport molaire (D)/(A) compris entre 0,1 et 10 environ. Le constituant (A) et l'agent (C) sont généralement dans un rapport molaire (C)/(A) inférieur ou égal à q, lorsque C est un acide ou un sel.

Parmi les métaux M couramment utilisables, on peut citer le fer, le nickel, le cobalt, le rhodium, le ruthénium, l'iridium, le palladium, le platine. Parmi les atomes ou molécules Z couramment utilisables, on peut citer le monoxyde de carbone, les halogènes, l'éthylène, le norbornadiène, le cyclooctadiène, l'acétylacétone. Enfin, le degré de coordination q peut être couramment compris entre 2 et 6 inclusivement selon le métal et/ou les ligands utilisés.

Tout composé organique ne possédant pas de centre d'asymétrie peut être soumis à une réaction de synthèse énantiosélective selon l'invention. Il en va ainsi de certaines oléfines, de certaines cétones, de certains diènes, éventuellement fonctionnalisés. Des exemples de réactions selon l'invention sont les réactions bien connues suivantes :

- hydrogénation et hydroformylation de substrats organiques insaturés tels que les oléfines éventuellement fonctionnalisées en $\alpha$ de la double liaison, en particulier les déshydroaminoacides ou leurs dérivés N-acétylés tels que les acides acétamidocarboxyliques ; les réactions d'hydrogénation sont généralement effectuées à une température comprise entre 0 et 200°C environ et sous une pression comprise entre 1 et 200 bars environ ; les réactions d'hydroformylation sont généralement effectuées à une température comprise entre 15 et 300°C environ et sous une pression comprise entre 1 et 350 bars environ ; ces réactions peuvent être appliquées industriellement notamment à la synthèse d'édulcorants non nutritifs comme l'aspartame et le (R)6-méthyltryptophane ;
- réduction de cétones et d'imines prochirales par des organosilanes, en particulier des monohydrosilanes et, de préférence, des dihydrosilanes (réaction d'hydrosilylation) ;
- cyclodimérisation et dimérisation des diènes conjugués, et codimérisation de l'éthylène et d'un diène conjugué; ces réactions sont généralement effectuées à une température comprise entre -30 et +100°C environ et sous une pression inférieure à 100 bars environ ;
- hydrogénation de composés carbonylés parmi lesquels on peut mentionner la quinuclidinone et son chlorhydrate, les $\alpha$-cétoamides, tels que le N-benzylbenzoylformamide ou le N-benzyl hydroxy-4 benzoylformamide, les composés $\alpha$-dicarbonylés comme l'oxo-2-diméthyl-3,3-butanolide-1,4 (encore dénommé cétopentoyllactone) et les $\alpha$-cétoesters tels que les esters benzoylformiques.

Les nouveaux ligands phosphorés chiraux selon l'invention produisent des résultats particulièrement avantageux dans l'hydrogénation asymétrique de composés carbonylés. Dans cette application, ils permettent d'améliorer significativement la vitesse et/ou le rendement optique de la réaction et/ou d'obtenir au choix l'une ou l'autre des configurations optiques du produit de la réaction. Par exemple, ils permettent, dans la réaction d'hydrogénation du N-benzylbenzoylformamide, d'obtenir la N-benzylmandélamide avec de bons rendements optiques après 1 à 3 heures à 20°C et, le cas échéant, dans sa configuration (R). De même, l'hydrogénation de l'oxo-2 diméthyl-3,3-butanolide-1,4 peut être réalisée avec un excès énantiomérique atteignant jusqu'à 95% après moins d'une heure de réaction et, le cas échéant, en atteignant la configuration (S) du produit hydrogéné.

La réaction d'hydrogénation de composés carbonylés est généralement effectuée à une température comprise entre -20°C et 200°C, de préférence entre 0° et 120°C environ, pendant une durée comprise entre 2 minutes et 4 heures environ, et sous une pression comprise entre 1 et 200 bars environ.

Par ailleurs, le rapport molaire du substrat (composé de départ) au métal M catalyseur peut atteindre des valeurs élevées de 5000, voire 10 000 ou davantage.

Pour l'obtention d'un meilleur excès énantiomérique, on peut mentionner, comme solvants, les solvants hydrocarbonés aromatiques, tels que le benzène, le toluène et le xylène, les alcools aliphatiques, tels que l'éthanol, et les éthers aliphatiques ou cycloaliphatiques, en particulier l'éther éthylique, le dioxanne et le tétrahydrofuranne, et les mélanges compatibles de ces solvants.

Les exemples suivants sont donnés à titre illustratif et non limitatif de la présente invention.

EXEMPLE 1

On fait réagir dans le tétrahydrofuranne sec, à une température de 70°C et sous atmosphère d'azote, 2 moles de chlorodicyclohexylphosphine avec 1 mole de 5-oxoprolinol (de pureté supérieure à 99%) pendant 72 heures en présence d'un excès de triéthylamine. On filtre le précipité de chlorhydrate de triéthylammonium qui se forme puis on évapore le benzène sous vide. On obtient alors 0,81 mole de ligand de pureté chimique évaluée à 95%, dont les données du spectre de résonance magnétique nucléaire du phosphore 31 sont rassemblées au tableau I.

EXEMPLE 2

On répète la méthode de synthèse de l'exemple 1 en remplaçant la chlorodicyclohexylphosphine par la chlorodicyclopentylphosphine. Dans ces conditions, on obtient 0,8 mole de ligand de pureté chimique évaluée à 98%, dont les données du spectre de résonance magnétique nucléaire du phosphore 31 sont rassemblées au tableau I.

EXEMPLE 3

On répète la méthode de synthèse de l'exemple 1 en remplaçant la chlorodicyclohexylphosphine par la chlorodiphénylphosphine. Dans ces conditions, on obtient 0,8 mole de ligand de pureté chimique évaluée à 99%, dont les données du spectre de résonance magnétique nucléaire du phosphore 31 sont rassemblées au tableau I.

EXEMPLE 4

On fait réagir, dans le toluène sec et à une température de 20°C, 1 mole du ligand préparé à l'exemple 1 (ci-après désigné L) et 0,5 mole de bis(chlorocyclooctadiènerhodium). La solution, laissée sous courant d'azote et sous agitation pendant 30 minutes, fonce. Le solvant est ensuite évaporé et on obtient, après séchage sous vide, 0,5 mole de complexe de formule $[RhClL]_2$. Ce complexe est identifié par son spectre de résonance magnétique nucléaire du phosphore 31, indiqué au tableau I.

EXEMPLE 5

On répète la méthode de synthèse de l'exemple 4 en remplaçant le ligand de l'exemple 1 par celui de l'exemple 2. Le complexe obtenu quantitativement est identifié par son spectre de résonance magnétique nucléaire du phosphore 31, indiqué au tableau I.

EXEMPLE 6

On répète la méthode de synthèse de l'exemple 4 en remplaçant le ligand de l'exemple 1 par celui de l'exemple 3. Le complexe obtenu quantitativement est identifié par son spectre de résonance magnétique nucléaire du phosphore 31, indiqué au tableau I.

Tableau I

| Exemple | 1 | 2 | 3 | 4 | 5 | 6 |
|---|---|---|---|---|---|---|
| δP-O | 150,4 | 145,5 | 117,0 | 163,3 | 139,4 | 164,8 |
| δP-N | 58,1 | 59,4 | 36,2 | 123,2 | 93,4 | 122,7 |

EXEMPLE 7

Après avoir introduit 0,012 mole d'oxo-2-diméthyl-3,3-butanolide-1,4 en solution dans 20 $cm^3$ de toluène, sous

atmosphère d'hydrogène, dans un réacteur en verre thermostaté à 50°C, on transfère dans le réacteur une solution de $3.10^{-5}$ mole du complexe obtenu à l'exemple 4 dans 10 $cm^3$ de toluène, et l'évolution de la réaction est suivie par lecture du volume d'hydrogène consommé. Après 120 minutes de réaction à 50°C, le solvant est évaporé et l'hydroxy-2-diméthyl-3,3-butanolide-1,4 de configuration (R) distillé sous vide. Le rendement est égal à 100%. Le pouvoir rotatoire spécifique $\alpha_D^{25}$ est mesuré sur une solution aqueuse de concentration 20,5 g/l. L'excès énantiomérique, calculé à partir du pouvoir rotatoire spécifique du produit pur (-50,7°), est égal à 95%.

EXEMPLE 8

On reproduit le processus opératoire de l'exemple 7 en remplaçant le complexe de l'exemple 4 par celui de l'exemple 5 et en effectuant l'hydrogénation à 20°C au lieu de 50°C. Le rendement en hydroxy-2-diméthyl-3,3-butanolide-1,4 de configuration (R) est égal à 100% après 70 minutes et l'excès énantiomérique est égal à 93%.

## Revendications

1. Ligands phosphorés chiraux comprenant au moins un radical dihydrocarbylphosphinoxy et au moins un radical dihydrocarbylphosphinamino, caractérisés en ce qu'ils sont choisis parmi ceux représentés par la formule :

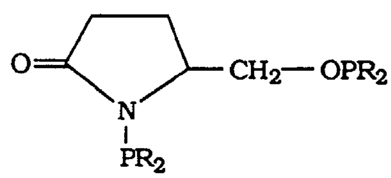

(III)

dans laquelle :

- R représente un radical hydrocarboné choisi parmi les radicaux alkyle linéaires ou ramifiés, cycloalkyle ou aryle.

2. Ligands phosphorés chiraux selon la revendication 1, caractérisés en ce que R est choisi parmi les radicaux isopropyle, cyclohexyle, cyclopentyle et phényle.

3. Procédé de fabrication de ligands phosphoré chiraux selon la revendication 1, de formule (III), caractérisé en ce qu'il consiste à faire réagir dans un solvant hydrocarboné, à une température comprise entre -50°C et 80°C et sous atmosphère de gaz inerte le 5-oxoprolinol avec au moins un composé de formule $P(R)_2$ Y, dans laquelle Y est choisi parmi les atomes d'halogène, ledit composé étant dans un rapport molaire, relativement au 5-oxoprolinol, supérieur ou égal à 2.

4. Procédé de fabrication selon la revendication 3, caractérisé en ce qu'on conduit la réaction en présence d'un excès d'amine tertiaire.

5. Application de ligands phosphorés chiraux selon l'une des revendications 1 et 2 à une réaction de synthèse énantiosélective de composés organiques, caractérisée en ce qu'elle consiste à faire réagir au moins un composé organique ne possédant pas de centre d'asymétrie avec (A) d'une part au moins un complexe de métal de transition de formule MZq dans laquelle M est un métal du groupe VIII de la Classification Périodique, q est le degré de coordination du métal M et Z est un atome ou une molécule capable de complexer le métal M et (B), d'autre part, au moins un tel ligand phosphoré chiral.

6. Application selon la revendication 5, caractérisée en ce que la réaction énantiosélective est choisie parmi l'hydrogénation et l'hydroformylation de substrats organiques insaturés, l'hydrosilylation de cétones et d'imines, la cyclodimérisation et la dimérisation de diènes conjugués, et la codimérisation de l'éthylène et d'un diène conjugué.

7. Application selon la revendication 5, caractérisée en ce que la réaction énantiosélective est l'hydrogénation de composés carbonylés.

**Patentansprüche**

1. Phosphorhaltige chirale Liganden enthaltend mindestens einen Dihydrocarbylphosphinoxy-Rest und mindestens einen Dihydrocarbylphosphinamino-Rest, dadurch gekennzeichnet, daß sie ausgewählt sind aus jenen der Formel:

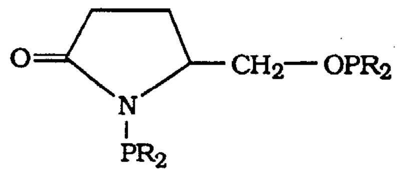

(III)

in der:

- R eine aus geradkettigen oder verzweigten Alkylgruppen, Cycloalkylgruppen oder Arylgruppen ausgewählte Kohlenwasserstoffgruppe bedeutet.

2. Phosphorhaltige chirale Liganden nach Anspruch 1, dadurch gekennzeichnet, daß R aus Isopropyl-, Cyclohexyl-, Cyclopentyl- und Phenylgruppen ausgewählt ist.

3. Verfahren zur Herstellung der phosphorhaltigen chiralen Liganden nach Anspruch 1 der Formel (III), dadurch gekennzeichnet, daß es darin besteht, 5-Oxoprolinol in einem Kohlenwasserstofflösungsmittel bei einer Temperatur zwischen -50°C und 80°C unter einer Inertgasatmosphäre mit mindestens einer Verbindung der Formel $P(R)_2Y$, in der Y aus Halogenatomen ausgewählt ist, wobei diese Verbindung in einem Molverhältnis bezogen auf 5-Oxoprolinol von größer oder gleich 2 eingesetzt wird, umzusetzen.

4. Verfahren nach Anspruch 3, dadurch gekennzeichnet, daß man die Reaktion in Gegenwart eines Überschusses eines tertiären Amins durchführt.

5. Verwendung der phosphorhaltigen chiralen Liganden nach einem der Ansprüche 1 und 2 bei einer enantioselektiven Synthesereaktion zur Herstellung von organischen Verbindungen, dadurch gekennzeichnet, daß sie darin besteht, mindestens eine organische Verbindung, die kein Asymmetriezentrum aufweist, mit (A) einerseits mindestens einem Übergangsmetallkomplex der Formel MZq, in der M ein Metall der Gruppe VIII des Periodensystems, q den Koordinationsgrad des Metalls Z und Z ein Atom oder Molekül, welches das Metall M zu komplexieren vermag, bedeuten, und (B) andererseits mindestens einem solchen phosphorhaltigen chiralen Liganden umzusetzen.

6. Verwendung nach Anspruch 5, dadurch gekennzeichnet, daß die enantioselektive Reaktion aus der Hydrierung und Hydroformylierung von ungesättigten organischen Substraten, der Hydrosilylierung von Ketonen und Iminen, der Cyclodimerisation und der Dimerisation von konjugierten Dienen und der Codimerisation von Ethylen und einem konjugierten Dien ausgewählt ist.

7. Verwendung nach Anspruch 5, dadurch gekennzeichnet, daß die enantioselektive Reaktion die Hydrierung von carbonylierten Verbindungen ist.

**Claims**

1. Chiral phospholigands comprising at least one dihydrocarbylphosphinoxy radical and at least one dihydrocarbylphosphinamino radical, characterised in that they are selected from those represented by the formula:

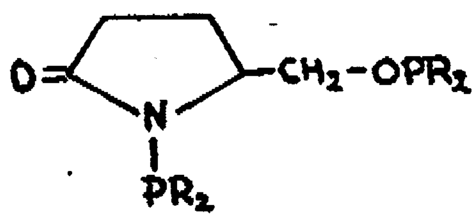

(III)

wherein:

R represents a hydrocarbon radical selected from the straight-chained or branched alkyl, cycloalkyl or aryl radicals.

**2.** Chiral phospholigands according to claim 1,
characterised in that R is selected from the isopropyl, cyclohexyl, cyclopentyl and phenyl radicals.

**3.** Process for producing chiral phospholigands according to claim 1, of formula (III), characterised in that it comprises reacting 5-oxoprolinol with at least one compound of formula $P(R)_2$ Y, wherein Y is selected from the halogen atoms, said compound being in a molar ratio greater than or equal to 2 relative to the 5-oxoprolinol, the reaction being carried out in a hydrocarbon solvent at a temperature between -50°C and 80°C, in an inert gas atmosphere.

**4.** Production process according to claim 3,
characterised in that the reaction is carried out in the presence of an excess of tertiary amine.

**5.** Use of chiral phospholigands according to one of claims 1 and 2 in an enantioselective reaction for synthesising organic compounds, characterised in that it comprises reacting at least one organic compound having no centre of asymmetry with (A), on the one hand, at least one transition metal complex of formula MZq wherein M is a metal of group VIII of the Periodic Table, q is the degree of coordination of the metal M and Z is an atom or molecule capable of complexing the metal M and (B), on the other hand, at least ones such chiral phospholigand.

**6.** Use according to claim 5, characterised in that the enantioselective reaction is selected from hydrogenation and hydroformylation of unsaturated organic substrates, hydrosilylation of ketones and imines, cyclodimerisation and dimerisation of conjugated dienes and codimerisation of ethylene and a conjugated diene.

**7.** Use according to claims 5, characterised in that the enantioselective reaction is the hydrogenation of carbonyl compounds.